# EUROPEAN PATENT SPECIFICATION

(11) **EP 2 128 239 B1**
(45) Date of publication and mention of the grant of the patent: **25.01.2012**
(21) Application number: 09158435.9
(22) Date of filing: 22.04.2009
(51) Int. Cl.: C12G 3/02, C12G 3/04

(54) **Alcoholic liquor additives and method for preparing the same**
Zusätze für alkoholische Getränke und Herstellungsverfahren dafür
Additifs de liqueur d'alcool et leur procédé de préparation

(30) Priority: 29.05.2008 KR 20080050400
(43) Date of publication of application: 02.12.2009
(73) Proprietor: Jang, Young-Sam, Mapo-Ku Seoul 121-210 (KR)
(72) Inventor: Jang, Young-Sam, Mapo-Ku Seoul 121-210 (KR)
(74) Representative: ABG Patentes, S.L.

(56) References cited:
- DATABASE WPI Week 200761, Derwent Publications Ltd., London, GB; AN 2007-646298, XP002534952 & KR 2007 0 025 584 A (KIM Y J) 08 March 2007
- DATABASE WPI Week 20031, Derwent Publications Ltd., London, GB; AN 2003-010731, XP002534922 & KR 2002 0 041 515 A (KIM H Y) 03 June 2002
- DATABASE WPI Week 200477, Derwent Publications Ltd., London, GB; AN 2004-781536, XP002534923 & KR 2004 0 067 551 A (JUNG I C) 30 July 2004
- DATABASE WPI Week 200218, Derwent Publications Ltd., London, GB; AN 2002-139859, XP002534886 & WO 02 00238 A1 (MEIJI MILK PROD CO LTD) 03 January 2002
- DATABASE WPI Week 200856, Derwent Publications Ltd., London, GB; AN 2008-J51291, XP002534924 & CN 101 168 026 A (WANG X) 30 April 2008
- DATABASE WPI Week 200271, Derwent Publications Ltd., London, GB; AN 2002-664105, XP002534953 & KR 2002 0 026 900 A (OH S G) 12 April 2002
- DATABASE WPI Week 199548, Derwent Publications Ltd., London, GB; AN 1995-366857, XP002534951 & CN 1 095 948 A (YANG K) 07 December 1994

## Description

### BACKGROUND OF THE INVENTION

### 1. Field of the Invention

The present invention relates to alcoholic liquor additives and a method for preparing the same, and more particularly, to an alcoholic liquor additive for improving flavor or taste of alcoholic liquors such as soju or Kaoliang wine, which is added to the liquors before drinking, thereby reducing difficulty or discomfort in drinking while curing a hangover or enabling fatigue recovery after drinking and a method for preparing the same.

### 2. Description of the Related Art

Drunk driving may take a person's life. About several hundred people are killed and about several thousand people are hurt in traffic accidents due to drunk driving each year. Drinking too much may not only cause material harm and mental injury to an individual, but also cause significant damages to other people. Therefore, wrong or bad drinking habits and/or culture may easily become a significant problem for society.

In order to minimize such damage caused by excessive drinking or bad drinking habits and/or culture, there may be a strong requirement for development of functional drinks or alcoholic liquor additives effective for curing a hangover after drinking, fatigue recovery, blood circulation, and so forth.

For instance, as for curing a hangover after drinking, a variety of commercial drinks containing herbal medicines (hereinafter referred to as 'herbal drink') are recently available in the market. Such herbal drinks are generally added to a high alcoholic wine or liquor with relatively low quality such as soju (an Korean rice wine) or Kaoliang wine (a Chinese wine) before drinking the wine.

Most herbal drinks include herbal ingredients for detoxification and/or for protection of a liver and, if the herbal drink is taken before or after drinking wine, a hangover from drinking may be more or less prevented.
KR2004067551 describes a method for manufacturing a cucumber alcoholic beverage with a reduced alcohol concentration thus reducing hangover after drinking the alcoholic beverage.
KR20070025584 discloses a method for preparing an alcoholic beverage from herbal medicines having blood nourishing effect, nutrition and robustness providing effect, to prevent hangover, alcoholic liver disease and neuronal diseases.
CN 1095948 describes an alcoholism-dispelling tea with certain protection effect on liver, kidney and other organs, consisting of tea and a mixture of herbal stuffs.
WO02/00238 discloses a composition for promoting alcohol metabolism or preventing sick feel from drinking and hagover, containing an alcohol fermentation product of citrus molasses and optionally a plant worm extract.
KR20020041515 describes a process of preparing a medicinal plant composition for the recovery of liver and kidney function as well as for the reduction of symptoms associated with alcohol intoxication and hangovers.
CN 101168026 discloses a traditional Chinese medicine for curing alcohol addiction without chemical sweetening agent and side effect.

However, owing to an inherent stimulant smell and taste of herbal substances, an original taste and/or flavor of a wine may be varied by adding the herbal substances to the wine. Therefore, there is a tendency to avoid addition of the herbal substances. Moreover, some drinks containing particular herbal materials may cause abdominal distension and/or nausea.

Furthermore, some drinks requiring expensive herbal medicines may not be desirable for blending with a cheap wine such as soju or Kaoliang wine.

### SUMMARY OF THE INVENTION

Accordingly, the present invention has been proposed to solve conventional problems described above and an object of the present invention is to provide an alcoholic liquor additive containing at least one natural material so as to cure a hangover after drinking or to facilitate fatigue recovery and/or blood circulation, characterized in that natural materials are used to reduce production costs of the additive.

Another object of the present invention is to provide a method for preparing the alcoholic liquor additive disclosed above.

In order to achieve the above objects of the present invention, there is provided a method for preparing an alcoholic liquor additive, comprising: preparing 18 to 22 wt.% of mulberry fruits, 8 to 12 wt.% of citron fruits, 4 to 6 wt.% of cucumber, 2 to 4 wt.% of cornus fruits, 2 to 4 wt.% of mulberry leaves, 1 to 2 wt.% of pine leaves, 15 to 20 wt.% of drinkable alcohol and the remainder being water relative to 100wt.% of additive; and placing the prepared mulberry fruits, citron fruits, cucumber, cornus fruits, mulberry leaves, pine leaves and drinkable alcohol in water and then ageing (often referred to as 'fermenting') the mixture at 10 to 15°C for 6 to 12 months.

The fermentation process described above may be performed in an earthenware jar.

The present invention also provides an alcoholic liquor additive prepared by the above preparation method.

As described above, the alcoholic liquor additive according to the present invention is obtained using various natural plants and/or fruits, thereby exhibiting beneficial effects on health such as hangover curing, fatigue recovery, blood circulation, and so forth.

Using natural materials may produce alcoholic liquor additives at low production costs as in KR20020026900A.

### BRIEF DESCRIPTION OF THE DRAWINGS

These and other objects, features, aspects, and advantages of the present invention will be more fully described in the following detailed description of preferred embodiments and examples, taken in conjunction with the accompanying drawings. In the drawings:
Fig. 1 is a configuration view illustrating a method for preparing an alcoholic liquor additive according to an exemplary embodiment of the present invention; and
Fig. 2 is a flow chart illustrating a process for preparation of an alcoholic liquor additive according to an exemplary embodiment of the present invention.

### DETAILED DESCRIPTION OF THE INVENTION

Hereinafter, an exemplary embodiment of the present invention will be described in greater detail with reference to the accompanying drawings.

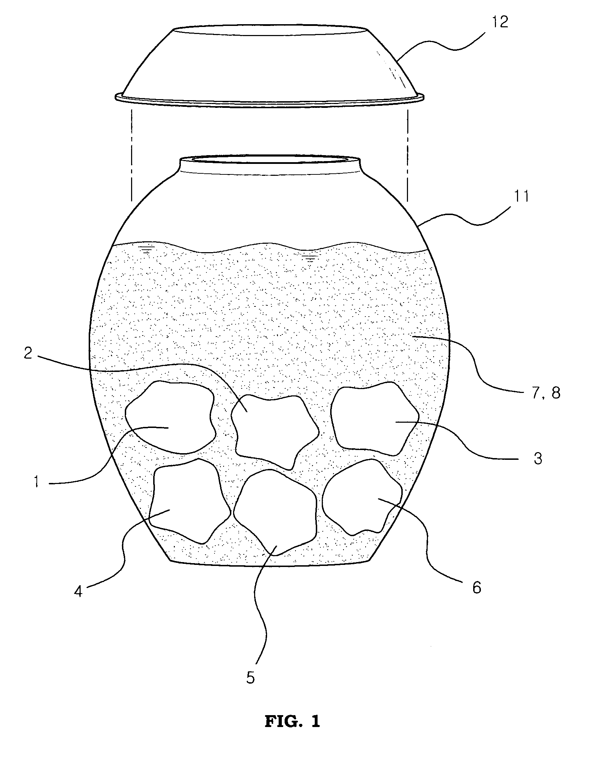

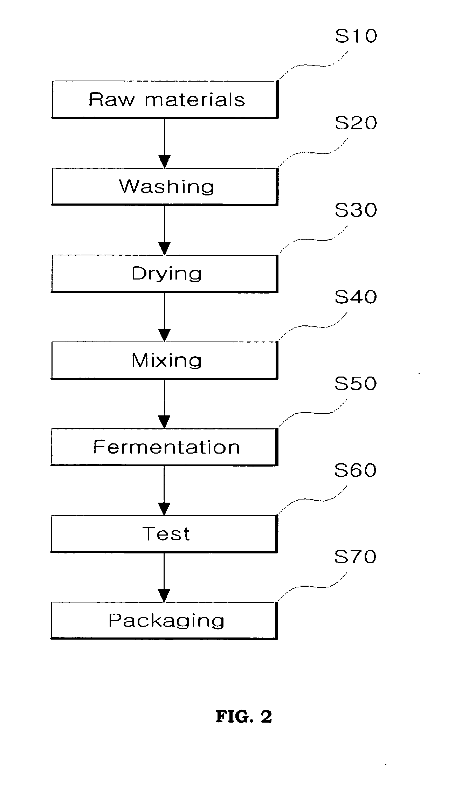

Fig. 1 is a configuration view illustrating a method for preparing an alcoholic liquor additive according to an exemplary embodiment of the present invention; and Fig. 2 is a flow chart illustrating a process for preparation of an alcoholic liquor additive according to an exemplary embodiment of the present invention.

As shown in Figs. 1 and 2, the alcoholic liquor additive according to the exemplary embodiment of the present invention comprises mulberry fruits 1, citron fruits 2, cucumber 3, cornus fruits 4, mulberry leaves 5, pine leaves 6, drinkable alcohol 7 and water 8, and is formed by fermenting a mixture of the above ingredients at room temperature for a desired period of time.

Mulberry fruit is a fruit of a mulberry tree named Morus sp. of Moraceae and is rich in glucose, fructose, calcium, iron, potassium, magnesium, zinc, vitamins A, B and/or C, thus being called 'the emperor of fruits.' The mulberry fruit has an iron content of at least about 4 to 5 times that of other fruits, a calcium content of at least double that of strawberry fruits, and a potassium content of at least double that of apple fruits.

The mulberry fruit also contains a lot of mineral ingredients such as magnesium, zinc, vitamins B and/or C, etc. and, especially, has a higher content of zinc than mulberry leaves, wherein zinc is an element liable to lacking in modern humans due to the influence of environmental hormones.

As a natural coloring matter, cyaniding-3-glucoside (C3G) enriched in the mulberry fruit is a functional compound with various physiological activities to exhibit stronger anti-ageing effects about 7 times that of tocopherol well known for its anti-ageing performance. Mulberry fruit also shows excellent efficacy for hypertension, diabetic disease, cure of hangovers, and the like.

The mulberry tree starts to bear bright green fruits in about April or May and the fruits are grown and ripen into a dark purplish black color in about June each year. The fully ripened fruits are harvested in June and prepared by washing the same.

If an amount of the mulberry fruits in the inventive additive is less than 18 wt.%, the efficacy of the mulberry fruit may be insufficient. On the other hand, if the amount exceeds 22 wt.%, a great amount of mineral ingredients in the mulberry fruit may cause digestive disorders. Therefore, a mulberry fruit ingredient is preferably added in an amount of 18 to 22 wt.% to the alcoholic liquor additive of the present invention.

Citron fruit is a fruit of a citron tree and frequently contains vitamin C as a main ingredient 3 times that of lemon. For this reason, citron fruit is beneficial for cold symptoms and skin cosmetic. The citron fruit is rich in organic acids and vitamin C abundant in the citron facilitates degradation of alcohol, thus removing alcohol residue in the body of a human and enabling cure of a hangover after drinking.

The citron fruit is also rich in vitamin B, carbohydrates and/or protein, as compared to other citrus fruits, The citron fruit also contains hesperidin capable of protecting capillaries, thereby enabling prevention of cerebrovascular disorders, strokes, etc. The citron fruit activates drainage and discharge functions of the body to exhaust wastes from the body, thus exhibiting superior effects in reinforcing gastrointestinal functions and/or blood circulation.

After clearly washing, the citron fruits are cut into desired pieces and prepared. If an amount of the citron fruits in the inventive additive is less than 8 wt.%, the efficacy of the citron fruit may be insufficient. On the other hand, if the amount exceeds 12 wt.%, it may cause a little strong sour taste or bitter taste, resulting in poor drinkable feel. Therefore, a citron ingredient is preferably added in an amount of 18 to 22 wt.% to the alcoholic liquor additive of the present invention.

Cucumber may have performance in alleviating edema disease and curing a hangover or drunkenness. Other symptoms caused by cardiogenic edema, renal edema or metabolic disturbance may also be reduced by the cucumber. Especially, a cucumber tea shows excellent effects on treatment of upset stomach or heartburn, vomiting and/or a severe headache after heavy drinking.

The cucumber for the alcoholic liquor additive is prepared by fully washing, peeling and cutting into pieces having a desired size. If an amount of the cucumber in the inventive additive is less than 4 wt.%, the efficacy of the cucumber may be insufficient. On the other hand, if the amount exceeds 6 wt.%, the cucumber may cause difficulty to a person with a cold and weak stomach. Therefore, a cucumber ingredient is preferably added in an amount of 4 to 6 wt.% to the alcoholic liquor additive of the present invention.

Cornus Officinalis (often referred to as 'cornus fruits') is a fruit of a cornus tree as a deciduous tree of Cornus controversa Hemsley. About March to April each year, yellow flowers firstly bloom before leaves thereof appear, and then, elongated round fruits grow and ripen reddish in about October. Such cornus fruits have an astringent taste and strong sour taste as well as slightly sweet taste, and are often harvested after the middle of October. The fruit is divided into pulpy substance and seeds and the pulpy substance may be used as a raw material of wine, tea and/or herbal medicines.

The cornus fruit includes glycosides such as cornin, morroniside, loganin, tannin, saponin, etc., organic acids such as tartaric acid, malic acid, grape acid, etc., and so forth. The fruit also contains vitamin A while being rich in sugar, thereby exhibiting enhanced blood circulation, strengthening sexual function and increasing an amount of sperm and/or bone marrow.

The cornus fruits are harvested and thoroughly washed, followed by naturally drying in the air and sun for one week. Then, after removing seeds, the fruits are again completely dried in the air and sun.

If an amount of the cornus fruits in the inventive additive is less than 2 wt.%, the efficacy of the fruit may be insufficient. On the other hand, if the amount exceeds 4 wt.%, sour taste may be too strong. Therefore, a cornus fruit ingredient is preferably added in an amount of 2 to 4 wt.% to the alcoholic liquor additive of the present invention.

Mulberry leaf is a leaf of a mulberry tree, sometimes called 'wizard's leaf.' Although the mulberry tree is generally cultivated to breed silkworms, root bark, fruits and/or leaves thereof are also widely used for preparation of herbal medicines. Especially, the mulberry leaf is rich in various mineral ingredients and contains 4.7 times more fibroin than a green tea leaf.

The mulberry leaf may reduce sugar content in blood of a diabetic patient, thus being helpful for prevention and/or treatment of diabetes. The mulberry leaf also has medical efficacy in alleviating asthma or cough, and reinforces eyesight. Rutin contained in the mulberry leaf strengthens blood vessels, especially, cerebrovascular tracts, while GABA ingredient depresses blood pressure and has efficacy in hypertension and/or arteriosclerosis. In addition, plenty of fibrin is included in the mulberry leaf so as to delay absorption of sugar into an intestinal canal, thus providing diuretic effects.

The mulberry leaf is harvested in the middle or later part of May when contents of useful ingredients are very high, and then, dried in the shade.

If an amount of the mulberry leaf in the inventive additive is less than 2 wt.%, the efficacy of the mulberry leaf may be insufficient. On the other hand, if the amount exceeds 4 wt.%, a high mineral content in the mulberry leaf may cause digestive disorder or malfunction. Therefore, a mulberry leaf ingredient is preferably added in an amount of 2 to 4 wt.% to the alcoholic liquor additive of the present invention.

Pine leaf is a leaf of a pine tree. The pine leaf is rich in carbohydrates and includes a variety of ingredients such as protein, fat, calcium, phosphorus, iron, vitamins A and/or C, etc., so as to exhibit beneficial effects on gastro-enteric disorders, hypertension, stroke, neuralgia, insomnia, anemia, asthma, drunkenness or intoxication, and the like. The pine leaf also contains many various organic acids which in turn are effective to prevent or treat feminine diseases such as leucorrhoea.

The pine leaf is rich in oxygen and minerals and, when a person feels tired during climbing, eating raw pine leaves may easily recover fatigue. In recent years, the pine leaf is increasingly being used as a food for beauty and health.

In addition, the pine leaf may stimulate body tissues including, for example, decrease of cholesterol level in blood or enlargement of peripheral nerves to promote hormone release, thus exhibiting beneficial effects on hypertension, myocardial infarction, etc. Moreover, the pine leaf may be useful for nervous stability and prevention and/or treatment of cold symptoms.

Among useful ingredients in the pine leaf, glucokinin has medical efficacy in diabetes, vitamin C and iron are effective to treat anemia, and some ingredients may remove nicotine poison as a harmful substance contained in cigarettes while reinforcing functions of digestive systems. Furthermore, the pine leaf has anti-ageing effects such as black hair growth stimulation, change in color of hair from white to black, enhanced hearing, etc.

The pine leaves are prepared by trimming, thoroughly washing with water and drying well in the sun.

If an amount of the pine leaf in the inventive additive is less than 1 wt.%, the efficacy of the pine leaf may be insufficient. On the other hand, if the amount exceeds 2 wt.%, tannins in the pine leaf may cause digestive trouble or malfunction in iron adsorption. Therefore, a pine leaf ingredient is preferably added in an amount of 1 to 2 wt.% to the alcoholic liquor additive of the present invention.

Drinkable alcohol may be so-called an 'alcohol spirit' as a source of wine. Raw materials of the alcohol spirit are generally classified into starch based material and carbohydrate based material. If the starch generally included in crops such as rice, barley, corn, etc. as well as sweet potato, potato, tapioca, and the like, undergoes saccharification using amylase or other enzymes to convert the starch into fermentable sugar ingredients, followed by employing a fermentation process, a drinkable alcohol may be produced.

The alcohol spirit is obtained by fermenting starch or sugar-containing raw materials and distilling the resulting product to have more than 85% alcohol concentration. The alcohol spirit shows aromatic properties and, when being absorbed in the body of a human, may act on the central nervous system or function as a tranquilizer, or may have a role of supplying calories to the human body as a food.

The alcohol spirit commonly means ethyl alcohol. Pure ethyl alcohol is a colorless, odorless and tasteless fluid type liquid, has unique aromatic properties and stimulating properties, and generates blue flame when burning the liquid.

The alcohol spirit with specified characteristics including intoxication properties, compatibility and/or solubility, may be employed in various applications such as medicaments and industrial products other than the drinkable alcohol. The intoxication properties of the alcohol spirit may be applied in manufacture of different wines such as diluted soju or cheongju (Japanese sake) as a filler, and so forth. The compatibility and the solubility of the alcohol spirit may be useful for edible materials, chemical engineering raw materials, reactants, leaching agents, precipitants, hygienic products, nutrients, and/or industrial additives.

If an amount of the drinkable alcohol in the inventive additive is less than 15 wt.%, constitutional ingredients of the additive may be insufficiently degraded to have difficulty in ageing, that is, fermentation thereof. On the other hand, if the amount exceeds 20 wt.%, alcohol concentration is too high so as to deteriorate drinkable feel of a wine including the additive. Therefore, a drinkable alcohol ingredient is preferably added in an amount of 15 to 20 wt.% to the alcoholic liquor additive of the present invention.

Lastly, water 8 is an essential material constituting about 70% of a human body and a certain amount of water must be supplied to the body everyday. It may be considered that the phenomena of life in terms of the human body is a complicated mechanism of chemical variations occurring due to different materials dissolved in water.

The water used herein may include clean and pure water such as first grade water, clean underground water, etc.

As shown in Fig. 1, the alcoholic liquor additive according to the exemplary embodiment of the present invention is produced by: preparing individual materials including mulberry fruits 1, citron fruits 2, cucumber 3, cornus fruits 4, mulberry leaves 5, pine leaves 6, drinkable alcohol 7 and water 8; placing a mixture of these materials into an earthenware jar 11; putting a lid 12 on the jar 11 to air-tightly seal the same; and fermenting the mixture at room temperature of 10 to 15°C for 6 to 12 months.

The earthenware jar 11 is traditionally used to contain and store seasonings, main and/or subsidiary foods, or drinks, and may function as a tool for wine fermentation. An earthenware product is fabricated by kneading earth to prepare a paste, slightly drying the paste in the shade, placing the slightly dried paste on a spinning wheel, shaping the paste in a desired form while rotating the spinning wheel, applying glaze to the shaped product, and heating the glaze coated product in a kiln.

The fabricated earthenware product has a number of fine pores on the surface thereof, through which air flows in and out the earthenware, so as to enable fresh preservation of crop foods, soybean sources or other seasonings and, in addition, to enable fermentation of alcoholic liquors or drinks.

If a fermentation temperature is lower than 10°C, constitutional ingredients of the additive may not be sufficiently released. On the other hand, if the temperature is higher than 15°C, the above ingredients may be deteriorated and cause reduction in efficacy thereof. Therefore, the fermentation is preferably performed at room temperature in the range of 10 to 15°C.

If a fermentation period is shorter than 6 months, constitutional ingredients of the additive may not be sufficiently released. On the other hand, if the period is longer than 12 months, the above ingredients may be deteriorated or taste of the additive may be deteriorated. Therefore, the fermentation is preferably performed during 6 to 12 months.

Referring to Fig. 2, a detailed description will be given of a method for preparing an alcoholic liquor additive according to an exemplary embodiment of the present invention.

20 wt.% of mulberry fruits, 10 wt.% of citron fruits, 5 wt.% of cucumber, 3 wt.% of cornus fruits, 3 wt.% of mulberry leaves, 1.5 wt.% of pine leaves, 17.5 wt.% of drinkable alcohol as raw materials and the remainder being water relative to 100wt.% of additive are prepared (S10).

Each of the prepared materials except the drinkable alcohol and the remainder water is thoroughly washed using water (S20), followed by drying the washed material in the sun or shade (S30). The dried materials as well as 17.5 wt.% of drinking alcohol are mixed and placed in an earthenware jar 11 (S40). After adding the remainder water to the jar 11 and putting a lid 12 on the jar to air-tightly seal the same, the sealed jar is subjected to fermentation at room temperature in the range of 10 to 15°C for 6 to 12 months (S50).

An ingredient test is performed for the fermented product, that is, the alcoholic liquor additive (S60). The additive having passed the test is then introduced into a sterilized container and then the container undergoes shipment and/ or distribution after packaging via a hygienic vacuum packaging apparatus (S70).

When a small amount of the additive produced as described above was added to a wine and the wine was subjected to a drinking test for determination of effect of the additive after drinking, test results are shown in the following tables.

### Effects of additive after drinking wine

A desired amount (for example, 60ml) of the alcoholic liquor additive according to the exemplary embodiment of the present invention was added to a commercial wine, that is, soju (alcohol content of 20 %, 300ml). For 120 adult men with an age distribution of 30 to 60 years who participated in the test, the prepared soju (360ml) was provided in an amount more than drink capacity of each of them, for example, 1 to 3 bottles per each person as shown in Table 1. After 10 minutes, effects of the alcoholic liquor additive were examined and results thereof are shown in Table 2.

**TABLE 1**

| Age distribution | 30 - 40 years | 41 - 50 years | 51 - 60 years | Total |
|---|---|---|---|---|
| Number of persons | 40 | 45 | 35 | 120 |

**TABLE 2**

| Drinking amount (number of wine bottles) | Number of persons | Excellent effect | Fair effect | Poor effect | Efficiency (%) |
|---|---|---|---|---|---|
| 1 | 55 | 30 | 25 | 0 | 100% |
| 2 | 45 | 31 | 14 | 0 | 100% |
| 3 | 20 | 12 | 8 | 0 | 100% |
| Total | 120 | 73 | 47 | 0 | |

From the above Table 2, the excellent effect means a condition in that the person has a clear consciousness sufficient to further drink, even after drinking soju with the alcoholic liquor additive of the present invention.

Likewise, the fair effect indicates a condition in that the person slightly becomes tipsy and feels giddy but has a clear consciousness sufficient to further drink, even after drinking soju with the alcoholic liquor additive of the present invention.

The poor effect is determined as a case where the person gets drunk and may not further drink, after drinking a soju with the alcoholic liquor additive of the present invention.

From test results as disclosed above, it was found that most persons participated in the test slightly feel the effects of drink only and may drink the wine more than usual quantity. Consequently, it may be demonstrated that the alcoholic liquor additive according to the exemplary embodiment of the present invention is effective to cure hangover after drinking.

Accordingly, the alcoholic liquor additive according to the exemplary embodiment of the present invention comprising mulberry fruits, citron fruits, cucumber and/or mulberry leaves in desired composition thereof may have efficacy in curing a hangover after drinking. Moreover, adding desired amounts of citron fruits and/or cornus fruits to the additive may enable improved blood circulation after drinking, while pine leaves may have effects on fatigue recovery after drinking.

## Claims

1. A method for preparing an alcoholic liquor additive, comprising:
preparing 18 to 22 wt.% of mulberry fruits, 8 to 12 wt.% of citron fruits, 4 to 6 wt.% of cucumber, 2 to 4 wt.% of cornus fruits, 2 to 4 wt.% of mulberry leaves, 1 to 2 wt.% of pine leaves, 15 to 20 wt.% of drinkable alcohol and the remainder being water relative to 100wt.% of additive; and
placing the prepared mulberry fruits, citron fruits, cucumber, cornus fruits, mulberry leaves, pine leaves and drinkable alcohol in water and then fermenting the mixture at 10 to 15°C for 6 to 12 months.

2. The method according to claim 1, wherein the fermentation process is performed in an earthenware jar.

3. An alcoholic liquor additive obtainable by the preparation process as set forth in claim 1 or 2.

## Patentansprüche

1. Verfahren zum Herstellen eines alkoholischen Spirituosen-Zusatzes, das umfasst:
Herstellen von 18 bis 22 Gew.-% von Maulbeer-Früchten, 8 bis 12 Gew.-% von Citron-Früchten, 4 bis 6 Gew.-% von Gurke, 2 bis 4 Gew.-% von Cornus-Früchten, 2 bis 4 Gew.-% von Maulbeer-Blättern, 1 bis 2 Gew.-% von Kiefern-Nadeln, 15 bis 20 Gew.-% von trinkbarem Alkohol und der Rest ist Wasser relativ zu 100 Gew.-% des Zusatzes; und
Platzieren der hergestellten Maulbeer-Früchte, Citron-Früchte, Gurke, Cornus-Früchte, Maulbeer-Blätter, Kiefern-Nadeln und des trinkbaren Alkohols in Wasser und dann Fermentieren der Mischung bei 10 bis 15°C für 6 bis 12 Monate.

2. Verfahren nach Anspruch 1, wobei das Fermentierungsverfahren in einem Steingut-Gefäß durchgeführt wird.

3. Ein alkoholischer Spirituosen-Zusatz, der durch das Herstellungsverfahren nach dem Anspruch 1 oder 2 erhältlich ist.

## Revendications

1. Méthode de préparation d'un additif pour liqueur alcoolisée, comprenant :
- la préparation de 18 à 22% en poids de mûres, 8 à 12% de cédrat, 4 à 6% en poids de concombre, 2 à 4% de baies de cornouiller, 2 à 4% en poids de feuilles de mûrier, 1 à 2% en poids d'aiguilles de pin, 15 à 20% en poids d'alcool buvable, le reste étant de l'eau pour 100% en poids d'additif, et
- le placement de la préparation de mûres, de cédrats, de concombre, de baies de cornouiller, de feuilles de mûrier, d'aiguilles de pin et d'alcool buvable dans de l'eau et ensuite mettre le mélange à fermenter entre 10 et 15°C pendant 6 à 12 mois.

2. Méthode selon la revendication 1, **caractérisée en ce que** le processus de fermentation est réalisé dans une jarre en terre cuite.

3. Additif pour liqueur alcoolisée pouvant être obtenu par le procédé de préparation tel qu'exposé dans la revendication 1 ou 2.
